# EUROPÄISCHE PATENTSCHRIFT

(11) **EP 1 343 446 B1**
(45) Veröffentlichungstag und Bekanntmachung des Hinweises auf die Patenterteilung: **15.06.2005**
(21) Anmeldenummer: 01271187.5
(22) Anmeldetag: 18.12.2001
(51) Int. Cl.: A61F 9/06, G02F 1/133

(54) **BLENDSCHUTZVORRICHTUNG FÜR SCHWEISSERSCHUTZMASKEN**
ANTIGLARE DEVICE FOR WELDING PROTECTIVE MASKS
SYSTEME ANTIEBLOUISSANT POUR MASQUES DE PROTECTION POUR SOUDEURS

(30) Priorität: 21.12.2000 CH 249400
(43) Veröffentlichungstag der Anmeldung: 17.09.2003
(73) Patentinhaber: OPTREL AG, 9630 Wattwil (CH)
(72) Erfinder: ACKERMANN, Emil, CH-9630 Wattwil (CH)
(74) Vertreter: Frei, Alexandra Sarah
(86) Internationale Anmeldenummer: PCT/CH2001/000724
(87) Internationale Veröffentlichungsnummer: WO 2002/049554

(56) Entgegenhaltungen:
- EP-A- 0 027 518
- WO-A-97/15256
- WO-A-99/12060
- FR-A- 2 530 039
- US-A- 4 155 122
- US-A- 4 620 322
- US-A- 5 959 705

## Beschreibung

Die Erfindung betrifft eine Blendschutzvorrichtung für Schweisserschutzmasken gemäss Oberbegriff von Patentanspruch 1 sowie ein Verfahren zur Steuerung einer solchen Blendschutzvorrichtung gemäss Oberbegriff von Anspruch 18. Derartige Blendschutzvorrichtungen sind aus dem Dokument EP-A-0 027 518 bekannt. Weitere Blendschutzvorrichtungen sind z.B. aus der WO98/57606 oder der EP 0 550 384 bekannt, wobei ein optischer Sensor zur Erfassung des Schweisslichts mittels einer elektronischen Schaltung einen aktiven elektro-optischen Schutzfilter so schaltet, dass bei Auftreten von Schweisslicht sofort eine vorgegebene Abdunklungsstufe (Schutzstufe) eingeschaltet wird und umgekehrt bei Stopp des Schweisslichts die Verdunkelung sofort wieder ausgeschaltet wird.

Solche moderne Blendschutzvorrichtungen, insbesondere als Sichtfenster für Schweisserschutzmasken, enthalten als aktives Filterelement typischerweise eine Flüssigkristallzelle, welche den Lichtdurchgang mehr oder weniger sperrt, sobald die von einem Sensor detektierte Lichtintensität eine vorgegebene Schwelle übersteigt. Eine elektronische Schaltung in der Blendschutzvorrichtung umfasst dabei eine Auswerteschaltung für das Sensorausgangssignal und eine Ansteuerschaltung für die Flüssigkristallzelle.

Zur Erfassung der Strahlung wurden bisher üblicherweise Silizium-Foto-Detektoren verwendet, welche die Strahlung in einem Wellenlängenbereich von Rot bis ins nahe Infrarot NIR, d.h. im Bereich z.B. von 600 - 900 nm erfassen. Jedes Schweissverfahren hat ein eigenes charakteristisches Lichtspektrum, das u.a. durch die Schweissparameter (Strom, Gas, Material) und das Schweissverfahren bestimmt wird. Bei praktisch allen Schweissverfahren ist der Anteil der emittierten Strahlung im UV Bereich gross, während im Rot- und NIR-Bereich ein relativ kleiner Strahlungsanteil abgegeben wird. Auf der andem Seite wird jedoch durch Fremdlicht, z.B. durch Kunstlicht, ein relativ grosser Anteil im langwelligen Bereich, d.h. im NIR- und Rot-Bereich, abgegeben, während der UV Anteil von Kunstlicht relativ gering ist. Deshalb wird im Empfindlichkeitsbereich der bisherigen Silizium-Detektoren ein relativ kleiner Anteil des Schweisslichts und dem gegenüber jedoch ein grosser Anteil von Fremd- und Störlicht erfasst, was die Unterscheidung des Schweisslichts von diesem Fremdlicht äusserst schwierig macht.

Um nun das Schweisslicht vom Fremdlicht und irgendwelchen Störlichteinflüssen unterscheiden zu können zur Ansteuerung des Schutzfilters, wird eine sehr aufwändige Elektronik benötigt. Insbesondere wird dabei das sogenannte Flackerlicht aus dem Schweisslichtbogen zur Separierung des Schweisslichts vom Fremdlicht verwendet. Bei ungenügender Unterscheidung des Schweisslichts vom Fremdlicht tritt ein Fehlverhaltens auf, d.h. dass die Abdunkelung nicht einschaltet bei auftretendem Schweisslicht, weil die Fremdlichtquellen überwiegen, bzw. umgekehrt, dass die Abdunkelung nicht aufgehoben wird, wenn das Schweisslicht abstellt. Bei heutigen Auswerteschaltungen werden deshalb sogenannte Flackerlichtschaltungen eingesetzt, die das Flackerlicht des Schweisslichts in einem bestimmten Frequenzbereich erfassen, ausfiltern und zur Auswertung verwenden. Das Signal-Rausch-Verhältnis ist hier jedoch sehr schlecht, so dass solche Flackerlichtschaltungen äusserst empfindlich, aufwändig und gegen Störungen anfällig sind.

Es ist deshalb Aufgabe der vorliegenden Erfindung, eine Blendschutzvorrichtung zu schaffen, welche diese bisherigen Nachteile überwindet, welche unempfindlicher ist gegen Einflüsse von Fremdlicht und welche eine einfachere, bessere und sicherere Steuerung der Schutzfilter ohne Fehlverhalten ermöglicht.

Diese Aufgabe wird erfindungsgemäss gelöst durch eine Blendschutzvorrichtung nach Anspruch 1, bzw. durch ein Verfahren zur Steuerung einer Blendschutzvorrichtung nach Anspruch 18. Mit dem UV-Detektor welcher eine Fluoreszenzschicht und einen zugeordneten Silizium Fotodetektor aufweist als optischem Sensor wird direkt und zum grossen Teil das effektive Schweisslicht erfasst, während entsprechend dem relativ geringeren UV-Anteil von Fremdlicht und Störlichteinflüssen deren Einfluss gering ist. Mit der Erfassung und Auswertung der gemessenen UV-Intensität durch eine entsprechende Auswerteschaltung zur Steuerung des Schutzfilters wird eine relativ einfache, unempfindliche und vor Fremdeinflüssen sichere Schaltung des Schutzfilters erreicht.

Insbesondere kann mit dieser erfindungsgemässen Vorrichtung und mit diesem Verfahren eine einfachere, sicherere und bessere elektronische Schaltung zur Steuerung der Schutzfilter eingesetzt werden. So kann z.B. auf eine aufwändige und sehr empfindliche Flackerlichterfassung und -auswertung in der elektronischen Schaltung verzichtet werden.

Die abhängigen Patentansprüche betreffen vorteilhafte Weiterbildungen der Erfindung mit weiteren Vorteilen bezüglich Einfachheit, Funktionssicherheit, optimaler Einstellbarkeit der Abdunkelung und universeller Anwendbarkeit sowie auch mit zusätzlichen Vorteilen in der Handhabung solcher Blendschutzvorrichtungen.

Im folgenden wird die Erfindung anhand von Beispielen und Figuren weiter erläutert, dabei zeigen:
- Fig.1: an einer spektralen Intensitätsverteilung die erfindungsgemässe Unterscheidung von Schweisslicht und Fremdlicht,
- Fig. 2: eine erfindungsgemässe Blendschutzvorrichtung mit UV-Detektor an einer Schweisserschutzmaske,
- Fig. 3: einen UV-Detektor mit Fluoreszenzschicht und Si-Fotodetektor,
- Fig.4: einen UV-Detektor mit Fluoreszenzschicht, Wellenleiter und Si-Fotodetektor,
- Fig. 5: einen separaten UV-Detektor mit Funksignalübertragung,
- Fig. 6: ein Beispiel einer einstellbaren Blendschutzvorrichtung an einer Schweisserschutzmaske.

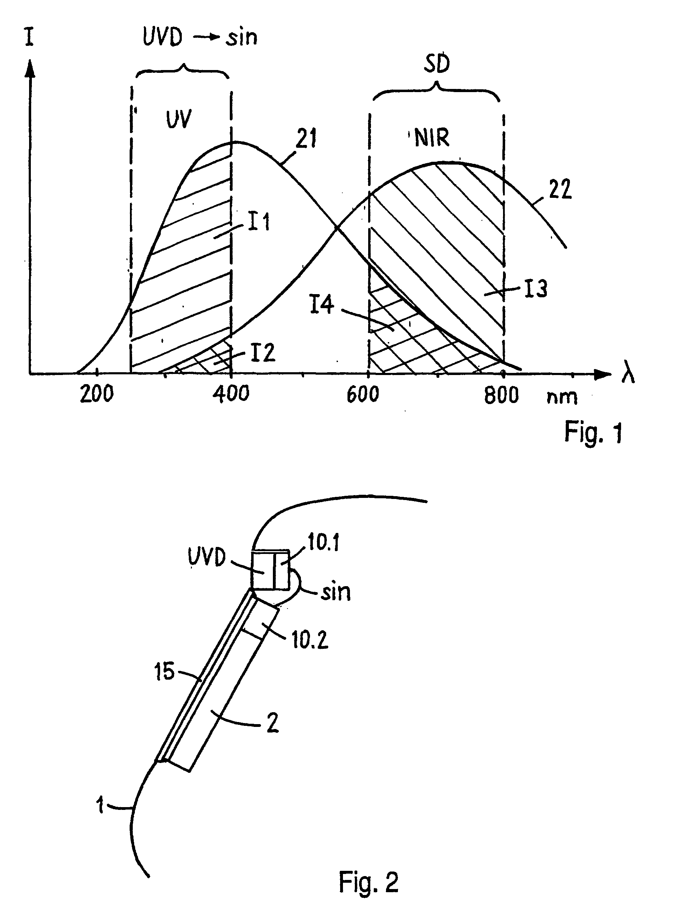

Fig. 1 zeigt schematisch die spektralen Intensitätsverteilungen I(λ) für ein Schweisslicht 21 mit einem hohen Intensitätsanteil im kurzwelligen Bereich und für eine Kunstlichtquelle als Fremdlicht 22 mit einem hohen Anteil im langwelligen Bereich. Das Schweisslicht weist einen hohen Intensitätsanteil 11 im UV Bereich auf, der von einem UV-Detektor UVD erfasst wird. Das Kunstlicht weist dagegen einen hohen Intensitätsanteil 13 im roten Bereich NIR auf, der von einem üblichen Si-Fotosensor SD erfasst wird. Im UV Bereich weist das Kunstlicht 22 einen kleinen Intensitätsanteil I2 auf, während umgekehrt das Schweisslicht 21 im NIR Bereich einen kleinen Intensitätsanteil I4 aufweist.

Der UV-Detektor UVD erfasst z.B. einen kurzwelligen Bereich von 250 - 400 nm und der Si-Fotodetektor SD einen langwelligen Bereich von z.B. 600 - 900 nm. Mit bisherigen Si-Sensoren SD wird somit ein relativ schlechtes Intensitätsverhältnis I4/I3 von Schweisslicht zu Fremdlicht erfasst, während mit dem erfindungsgemässen UV-Detektor UVD ein besonders günstiges Intensitätsverhältnis I1/I2 zur Auswertung erfasst werden kann.

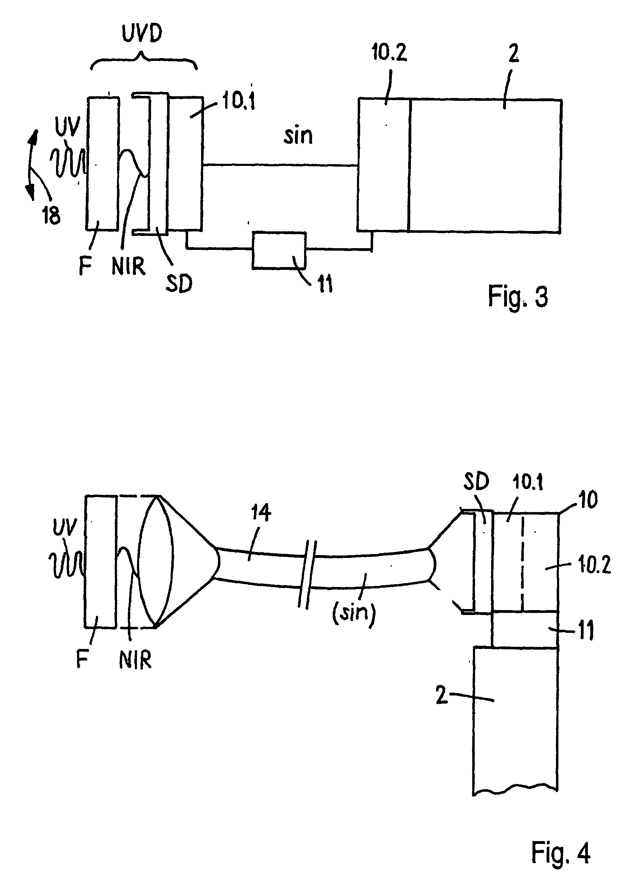

Die Erfindungsidee liegt darin, mit einfachen Mitteln das Schweisslicht möglichst direkt zu erfassen, so dass es wenig von Fremdlichteinflüssen überlagert wird und dazu einen spektral selektiven Fotodetektor in einem besonders günstigen Spektralbereich einzusetzen und mit dieser gezielten Erfassung des Schweisslichts die Abdunklung des Schutzfilters einfach, sicher und optimal zu steuern. Dazu dient ein UV-Detektor welcher eine Fluoreszenzschicht F und einen zugeordneten Si-Fotodetektor SD umfasst, wie dies zu Fig. 3 beschrieben ist.

Fig. 2 zeigt eine erfindungsgemässe Blendschutzvorrichtung an einer Schweisserschutzmaske 1 mit einem aktiven elektro-optischen Schutzfilter 2. Als optischer Sensor dient hier ein UV-Detektor UVD, der in einer Sensor Auswerteschaltung 10.1 ein der UV Intensität entsprechendes Signal "sin" zur Steuerung des Schutzfilters 2 erzeugt.

Die spektrale Empfindlichkeit des UV-Detektors ist vorzugsweise so ausgelegt, dass Strahlung in einem Wellenlängenbereich von z.B. 200 - 400 nm oder auch von 250 - 400 nm detektiert wird.

Fig. 3 zeigt den Aufbau eines erfindungsgemässen UV-Detektors, welcher aus einer Fluoreszenzschicht F und einem zugeordneten Si-Fotodetektor SD besteht. Die Fluoreszenzschicht weist eine Absorption im UV Bereich und eine Emission im langwelligen Bereich entsprechend der spektralen Empfindlichkeit des Si-Fotodetektors SD auf. Die Absorption der Fluoreszenzschicht F liegt beispielsweise im Bereich von 200 - 400 nm und die Emission im Bereich von 600 - 800 nm oder von 600 - 900 nm. D.h. die Fluoreszenzschicht ist so gewählt, dass das kurzwellige UV Schweisslicht in langwelliges, einfach zu detektierendes NIR Licht umgewandelt wird, welches von üblichen bekannten Si-Fotodetektoren erfasst werden kann. Die Sensor Auswerteschaltung 10.1 erzeugt dabei wieder ein Signal sin, das der detektierten UV Intensität entspricht, und mit dem durch eine einfache Ansteuerschaltung 10.2 das Schutzfilter 2 entsprechend diesem Signal sin des UV-Detektors gesteuert werden kann. Dies ermöglicht eine einfache, unempfindliche elektronische Schaltung 10, welche keine aufwändige und heikle bisherige Flackerlicht-Erfassung und -Auswertung im NIR Bereich benötigt. Anderseits kann aber eine Flackerlicht-Erfassung im UV Bereich dank günstigen Intensitäts-verhältnissen bzw. gutem Signal/Rausch Verhältnis auch viel einfacher realisiert werden. Die ganze Steuerung des Schutzfilters besteht somit aus den Teilschaltungen 10.1 und 10.2, welche auch in einer elektronischen Schaltung 10 zusammengefasst werden können. Dank der erfindungsgemässen besseren Erfassung des Schweisslichts könnte dieses Verfahren nicht nur bei elektrischen Schweissverfahren sondern z.B. auch beim Gasschweissen eingesetzt werden.

Die Ausführungen des UV-Detektors UVD als separate Einheit ermöglicht es, auch den UV Sensor, bzw. hier die Fluoreszenzschicht F, von der Blendschutzkassette örtlich zu trennen und damit optimal platzieren und ausrichten zu können.

Ein Ausführungsbeispiel dazu zeigt Fig. 4. Der UV-Detektor wird hier gebildet durch die Fluoreszenzschicht F mit einer Optik als Eingang in einen Glasfaser-Wellenleiter 14, welcher die transformierte NIR Strahlung an den Si-Detektor SD übermittelt (während sich Wellenleiter für die Übermittlung von UV Strahlung nicht eignen). Dieser separate UV Sensor benötigt keine eigene Energieversorgung. Die ganze elektronische Schaltung (10.1 und 10.2) mit der erforderlichen Stromversorgung 11 ist an der Blendschutzkassette angebracht.

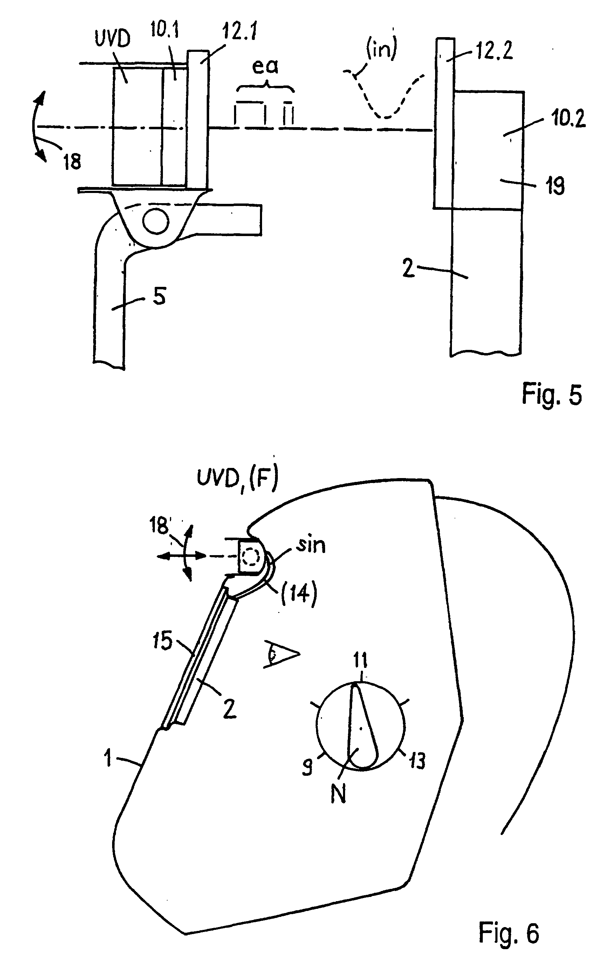

Fig. 5 zeigt eine weitere Möglichkeit, den UV-Detektor UVD örtlich von der Blendschutzkassette zu trennen. Diese Ausführung besteht aus zwei Teilen, die über Funk (RF) miteinander verbunden sind. Den ersten Teil bildet der UV-Detektor UVD mit der Sensor Auswerteschaltung 10.1 und mit einem RF Sender 12.1. Den zweiten Teil bildet ein RF Empfänger 12.2 mit einer Ansteuerschaltung 10.2 für das Schutzfilter 2. Mit dieser Ausführung kann vom RF Senderteil 12.1 ein der detektierten UV Intensität entsprechendes Intensitätssignal "in" erzeugt und über Funk an die Blendschutzkassette gesendet werden. In einer weiteren Ausführungsvariante kann am Sendeteil mittels einer entsprechenden Signalauswerteschaltung 10.1 auch nur ein einfaches ein/aus Signal "ea" generiert werden, welches leicht zu übermitteln ist und welches am RF Empfänger 12.2 die Abdunklung des Schutzfilters 2 ein- und ausschaltet.

Wie diese Beispiele illustrieren, kann der erfindungsgemässe UV-Detektor UVD auch ausserhalb der Schutzmaske angeordnet werden, insbesondere näher an der Schweissstelle, z.B. am Schweissgerät bzw. an dessen Handschutz 5. Hier ist die Intensität des detektierten Schweisslichts wesentlich höher als an der Schutzmaske und der Fremdlichteinfluss ist verhältnismässig entsprechend kleiner. Dies verbessert wiederum die Steuerung des Schutzfilters 2.

Wenn der UV-Detektor separat angeordnet ist und unterschiedliche Abstände zwischen Schweisslicht und Auge bzw. zwischen Schweisslicht und UV-Detektor auftreten, so können diese Unterschiede mit einer Abstandskompensation 19 in der elektronischen Schaltung 10 berücksichtig werden.

Bisher erfolgte die Abdunkelung auf eine fest vorgegebene bzw. fest eingestellte Schutzstufe N, d.h. die Transmission des Schutzfilters 2 wird dabei auf einen festen, der Schutzstufe entsprechenden tiefen Wert reduziert. Dank der erfindungsgemässen genaueren Erfassung des Schweisslichts kann nun aber auch eine automatische Steuerung der Abdunklung, d.h. eine Steuerung des Transmissionsgrads des Schutzfilters in Funktion der detektierten UV Strahlungsintensität, realisiert werden. Dabei kann z.B. ein vorgebbarer minimaler Schutzstufenwert Nmin eingestellt werden und über diesen hinaus in einem Variationsbereich von z.B. zwei bis drei weiteren Schutzstufen die Transmission reduziert nachgesteuert werden, also z.B. eine eingestellte minimale Schutzstufe Nmin = 9 und reduzierte Nachsteuerung in einem Bereich von N = 9 - 12, wobei die Gewichtung der UV Intensität für diese Steuerung der Schutzfilter-Transmission im Prinzip wählbar sein kann.

Fig. 6 zeigt eine Schweisserschutzmaske 1 mit einer Blendschutzvorrichtung, die einen separaten, ausrichtbaren UV-Detektor UVD aufweist. Diese Ausrichtbarkeit ist mit den Pfeilen 18 angezeigt (wie auch in Fig. 5 oder Fig. 3). Der UV-Detektor ist hier oberhalb des Schutzfilters 2 separat am Helm befestigt und z.B. über einen Wellenleiter 14 mit der Blendschutzkassette bzw. dem Schutzfilter 2 verbunden. Wichtig ist, dass der UV Detektor ausserhalb einer Vorsatzscheibe 15 angeordnet ist (wie auch im Beispiel von Fig. 2). Damit kann eine UV Absorption durch die Vorsatzscheibe 15, welche z.B. aus Polycarbonat besteht, die Funktion des UV-Detektors nicht beinträchtigen. Das Beispiel von Fig. 6 zeigt auch eine an sich bekannte, zusätzliche, von Hand einstellbare Schutzstufenverstellung, z.B. in einem Bereich von N = 9 - -13, welche in Kombination mit der erfindungsgemässen Schutzfiltersteuerung weitere Anwendungsvorteile ergibt.

Im Rahmen dieser Anmeldung werden die folgenden Bezeichnungen verwendet:
- 1: Schweisser-Schutzmaske
- 2: aktives elektro-optisches Schutzfilter (Blendschutzkassette)
- 5: Handschutz, Schweissgerät
- 10: elektronische Schaltung
- 10.1: Sensor Auswerteschaltung
- 10.2: Ansteuerschaltung für 2
- 11: Stromversorgung
- 12.1: RF-Sender
- 12.2: RF-Empfänger
- 14: Glasfaser Wellenleiter
- 15: Vorsatzscheibe für 2
- 18: ausrichtbar, einstellbar
- 19: Abstandskompensation
- 21: Schweisslicht
- 22: Kunstlicht, Fremdlicht
- N: Schutzstufenwert
- UVD: UV-Strahlungsdetektor
- F: Fluoreszenzschicht
- SD: Silizium (Si) Fotodetektor
- I: Lichtintensität
- λ: Wellenlänge
- UV: UV Bereich, kurzwellig
- NIR: NIR- und Rot-Bereich, langwellig
- sin: Intensitätssignal
- ea: Funksignal: ein/aus
- in: Funksignal: Intensität

## Patentansprüche

1. Blendschutzvorrichtung für eine Schweisserschutzmaske mit einem aktiven elektro-optischen Schutzfilter (2), einer elektronischen Schaltung (10) und einem optischen Sensor, welcher
als UV-Detektor (UVD) ausgebildet ist mit einer Sensor Auswerteschaltung (10.1), welche ein der detektierten UV Intensität entsprechendes Signal zur Steuerung des Schutzfilters (2) erzeugt, **dadurch gekennzeichnet,**
**dass** der UV-Detektor eine Fluoreszenzschicht (F) und einen zugeordneten Silizium Fotodetektor (SD) aufweist, wobei die Fluoreszenzschicht eine Absorption im UV Bereich und eine Emission im langwelligen Bereich, entsprechend der spektralen Empfindlichkeit des Si-Fotodetektor (SD), aufweist.

2. Blendschutzvorrichtung nach Anspruch 1, **dadurch gekennzeichnet, dass** der UV-Detektor (UVD) Strahlung in einem Wellenlängenbereich von 200 - 400 nm detektiert.

3. Blendschutzvorrichtung nach Anspruch 1, **dadurch gekennzeichnet, dass** die Absorption der Fluoreszenzschicht (F) im Bereich von 200 - 400 nm und die Emission im Bereich von 600 - 800 nm liegt.

4. Blendschutzvorrichtung nach Anspruch 1, **dadurch gekennzeichnet, dass** die Fluoreszenzschicht (F) über einen Glasfaser-Wellenleiter (14) mit dem Si-Fotodetektor (SD) verbunden ist.

5. Blendschutzvorrichtung nach einem der vorangehenden Ansprüche, **dadurch gekennzeichnet, dass** die elektronische Schaltung (10) eine Ansteuerschaltung (10.2) aufweist, welche das Signal des UV-Detektors zur Steuerung des Schutzfilters (2) verwendet.

6. Blendschutzvorrichtung nach einem der vorangehenden Ansprüche, **dadurch gekennzeichnet, dass** die elektronische Schaltung (10) keine Flackerlicht-Erfassung und -Auswertung enthält.

7. Blendschutzvorrichtung nach einem der vorangehenden Ansprüche, **dadurch gekennzeichnet, dass** der UV-Detektor (UVD) eine Sensor Auswerteschaltung (10.1) mit einem RF-Sender (12.1) aufweist und dass, davon getrennt, an der Schweisserschutzmaske (1) ein RF-Empfänger (12.2) mit einer Ansteuerschaltung (10.2) für das Schutzfilter (2) angeordnet ist.

8. Blendschutzvorrichtung nach Anspruch 7, **dadurch gekennzeichnet, dass** am RF-Sender (12.1) ein ein/aus Funksignal erzeugt wird.

9. Blendschutzvorrichtung nach Anspruch 7, **dadurch gekennzeichnet, dass** am RF-Sender (12.1) ein Intensitätssignal erzeugt wird.

10. Blendschutzvorrichtung nach einem der vorangehenden Ansprüche, **dadurch gekennzeichnet, dass** der UV-Detektor (UVD) an der Schutzmaske (1) ausserhalb einer Vorsatzscheibe (15) angeordnet ist.

11. Blendschutzvorrichtung nach einem der vorangehenden Ansprüche, **dadurch gekennzeichnet, dass** der UV-Detektor (UVD) ausserhalb der Schutzmaske (1) angeordnet ist.

12. Blendschutzvorrichtung nach einem der vorangehenden Ansprüche, **dadurch gekennzeichnet, dass** der UV-Detektor (UVD) an einem zugeordneten Schweissgerät (5) bzw. Handschutz angeordnet ist.

13. Blendschutzvorrichtung nach einem der vorangehenden Ansprüche, **dadurch gekennzeichnet, dass** der UV-Detektor (UVD) auf das Schweisslicht ausrichtbar bzw. einstellbar (18) angeordnet ist.

14. Blendschutzvorrichtung nach einem der vorangehenden Ansprüche, **dadurch gekennzeichnet, dass** bei unterschiedlichen Abständen Schweisslicht - Auge und Schweisslicht - UV-Detektor in der elektronischen Schaltung (10) eine Abstandskompensation (19) vorgesehen ist.

15. Blendschutzvorrichtung nach einem der vorangehenden Ansprüche, **dadurch gekennzeichnet, dass** eine automatische Steuerung der Abdunklung des Schutzfilters (2) in Funktion der detektierten UV-Strahlungsintensität vorgesehen ist.

16. Blendschutzvorrichtung nach Anspruch 15, **dadurch gekennzeichnet, dass** die Abdunklung mindestens einen vorgebbaren Schutzstufenwert einstellt und darüber hinaus in einem Variationsbereich von z.B. 2 - 3 Schutzstufen reduziert nachsteuert.

17. Blenäschutzvorrichtung nach einem der vorangehenden Ansprüche, **dadurch gekennzeichnet, dass** zusätzlich eine von Hand einstellbare Schutzstufeneinstellung (N) vorgesehen ist.

18. Verfahren zur Steuerung einer Blendschutzvorrichtung einer Schweisserschutzmaske mit einem aktiven elektro-optischen Filter (2) und einer elektronischen Schaltung (10), wobei UV-Licht mit einem optischen UV-Detektor (UVD) erfasst wird und mit einer Sensor-Auswerteschaltung (10.1) ein der UV Intensität entsprechendes Signal (sin) erzeugt und zur Steuerung des Schutzfilters (2) verwendet wird, **dadurch gekennzeichnet, dass** als UV-Detektor eine Fluoreszenzschicht (F) und ein zugeordneter Silizium Fotodetektor (SD) eingesetzt wird, wobei die Fluoreszenzschicht eine Absorption im UV Bereich und eine Emission im lang welligen Bereich, entsprechend der spektralen Empfindlichkeit des Si-Fotodetektor (SD), aufweist.

## Claims

1. Glare protection device for a welding protective mask with an active electro-optical protective filter (2), an electronic circuit (10) and an optical sensor, which is designed as an UV - detector (UVD) with a sensor evaluation circuit (10.1), which generates a signal for the controlling of the protective filter (2), which corresponds to the detected UV intensity, **characterised in that** the UV-detector comprises a fluorescent layer (F) and an assigned silicon photo detector (SD), wherein the fluorescent layer comprises an absorption in the UV range and an emission in the long-wave range corresponding to the spectral sensitivity of the Si - photo detector (SD).

2. Glare protection device according to claim 1. **characterised in that** the UV- detector (UVD) detects radiation in a wave-length range of 200 - 400 nm.

3. , Glare protection device according to claim 1, **characterised in that** the absorption of the fluorescent layer (F) lies within a range of 200 - 400 nm and the emission within a range of 600 - 800 run.

4. Glare protection device according to claim 1, **characterised in that** the fluorescent layer (F) is connected with the Si - photo detector (SD) through a fibre-glass wave guide (14).

5. Glare protection device according to one of the preceding claims, **characterised in that** the electronic circuit (10) comprises a driving circuit (10.2), which utilises the signal of the UV - detector for controlling the protective filter (2).

6. Glare protection device according to one of the preceding claims, **characterised in that** the electronic circuit (10) does not comprise a flickering light detection and - evaluation system.

7. Glare protection device according to one of the preceding claims, **characterised in that** the UV - detector (UVD) comprises a sensor evaluation circuit (10.1) with an RF - transmitter (12.1) and that, separate from it, on the welding protective mask (1) an RF - receiver (12.2) with a driving circuit (10.2) for the protective filter (2) is arranged.

8. Glare protection device according to claim 7, **characterised in that** in the RF-transmitter (12.1) a wireless on/off signal is generated.

9. Glare protection device according to claim 7, **characterised in that** in the RF-transmitter (12.1) an intensity signal is generated.

10. Glare protection device according to one of the preceding claims, **characterised in that** the UV - detector (UVD) is situated on the protective mask (1) outside a protection glass (15).

11. Glare protection device according to one of the preceding claims, **characterised in that** the UV - detector is arranged outside the protective mask (1).

12. Glare protection device according to one of the preceding claims, **characterised in that** the UV - detector (UVD) is arranged on an assigned welding device (5) or hand protection.

13. Glare protection device according to one of the preceding claims, **characterised in that** the UV - detector (UVD) is capable of being aligned or adjusted to the welding light (18).

14. Glare protection device according to one of the preceding claims, **characterised in that** in the case of differing distances between the welding light and the eye and the welding light and the UV - detector in the electronic circuit (10) a distance compensation (19) is provided.

15. Glare protection device according to one of the preceding claims, **characterised in that** an automatic controlling of the blacking out of the protective filter (2) in function of the detected UV radiation intensity is provided.

16. Glare protection device according to claim 15, **characterised in that** the blacking out sets at least a pre-definable protection level value and over and above this comprises a reduced follow-up control within a variation range of, e.g., 2 - 3 protection levels.

17. Glare protection device according to one of the preceding claims, **characterised in that** in addition a manual protection level setting (N) is provided.

18. Method for the controlling of the glare protection device of a welding protective mask with an active electro-optical filter (2) and an electronic circuit (10), wherein UV light is detected with an optical UV - detector (UVD) and that with a sensor evaluation circuit (10.1.) a signal (sin) corresponding to the UV intensity is generated and made use of for the controlling of the protective filter (2), **characterised in that** a fluorescent layer (F) and an assigned silicon photo detector (SD) is used as the UV - detector, wherein the fluorescent layer comprises an absorption in the UV range and an emission in the long-wave range corresponding to the spectral sensitivity of the Si - photo detector (SD).

## Revendications

1. Dispositif anti-éblouissement pour un masque de protection de soudeur avec un filtre protecteur électro-optique actif (2), un circuit électronique (10) et un capteur optique qui est conçu comme un détecteur d'UV (UVD) avec un circuit d'évaluation de capteur (10.1) qui génère un signal de commande du filtre de protection (2) correspondant à l'intensité des UV détectés, **caractérisé en ce que** le détecteur d'UV présente une couche fluorescente (F) et un photodétecteur au silicium (SD) associé, la couche fluorescente absorbant le rayonnement dans la plage des UV et émettant dans la plage des ondes longues en fonction de la sensibilité spectrale du photodétecteur au silicium (SD).

2. Dispositif anti-éblouissement selon la revendication 1, **caractérisé en ce que** le détecteur d'UV (UVD) détecte le rayonnement dans une plage de longueurs d'onde allant de 200 à 400 nm.

3. Dispositif anti-éblouissement selon la revendication 1, **caractérisé en ce que** la couche fluorescente (F) absorbe dans une plage comprise entre 200 et 400 nm et émet entre 600 et 800 nm.

4. Dispositif anti-éblouissement selon la revendication 1, **caractérisé en ce que** la couche fluorescente (F) est reliée au photodétecteur au silicium (SD) par un guide d'ondes en fibres de verre (14).

5. Dispositif anti-éblouissement selon l'une des revendications précédentes, **caractérisé en ce que** le circuit électronique (10) comprend un circuit de déclenchement (10.2) qui utilise le signal du détecteur d'UV pour commander le filtre de protection (2).

6. Dispositif anti-éblouissement selon l'une des revendications précédentes, **caractérisé en ce que** le circuit électronique (10) ne comprend pas de détection ni d'évaluation des scintillements lumineux.

7. Dispositif anti-éblouissement selon l'une des revendications précédentes, **caractérisé en ce que** le détecteur d'UV (UVD) comprend un circuit d'interprétation du capteur (10.1) avec un émetteur de fréquences radio (12.1) et **en ce qu'**il est prévu, séparément, sur le masque de protection du soudeur (1), un récepteur de fréquences radio (12.2) avec un circuit de déclenchement (10.2) pour le filtre de protection (2).

8. Dispositif anti-éblouissement selon la revendication 7, **caractérisé en ce qu'**un signal radio marche/arrêt est produit par l'émetteur de fréquences radio (12.1).

9. Dispositif anti-éblouissement selon la revendication 7, **caractérisé en ce qu'**un signal d'intensité est généré par l'émetteur de fréquences radio (12.1).

10. Dispositif anti-éblouissement selon l'une des revendications précédentes, **caractérisé en ce que** le détecteur d'UV (UVD) est disposé sur le masque de protection (1) à l'extérieur d'une vitre supplémentaire (15).

11. Dispositif anti-éblouissement selon l'une des revendications précédentes, **caractérisé en ce que** le détecteur d'UV (UVD) est disposé à l'extérieur du masque de protection (1).

12. Dispositif anti-éblouissement selon l'une des revendications précédentes, **caractérisé en ce que** le détecteur d'UV (UVD) est disposé sur un appareil de soudage (5) ou une protection des mains associés.

13. Dispositif anti-éblouissement selon l'une des revendications précédentes, **caractérisé en ce que** le détecteur d'UV (UVD) est disposé de manière orientable ou ajustable (18) vers la lumière de soudage.

14. Dispositif anti-éblouissement selon l'une des revendications précédentes, **caractérisé en ce qu'**une compensation de la distance (19) est prévue dans le circuit électronique (10) lorsque les distances entre la lumière de soudage et l'oeil et entre la lumière du soudage et le détecteur d'UV sont différentes.

15. Dispositif anti-éblouissement selon l'une des revendications précédentes, **caractérisé en ce qu'**il est prévu une commande automatique de l'obscurcissement du filtre de protection (2) en fonction de l'intensité du rayonnement ultraviolet détectée.

16. Dispositif anti-éblouissement selon la revendication 15, **caractérisé en ce que** la commande d'obscurcissement ajuste au moins une valeur de niveau de protection prédéterminée et l'ajuste ensuite de manière limitée dans une plage de variation de 2-3 niveaux de protection par exemple.

17. Dispositif anti-éblouissement selon l'une des revendications précédentes, **caractérisé en ce qu'**il est prévu en outre un réglage du niveau de protection (N) réglable manuellement.

18. Procédé pour la commande d'un dispositif de protection anti-éblouissement d'un masque de soudeur avec un filtre électro-optique actif (2) et un circuit électronique (10), dans lequel une lumière ultraviolette est captée par un détecteur d'UV (UVD) et un signal (sin) correspondant à l'intensité des UV est produit avec un circuit d'interprétation du capteur (10.1) et utilisé pour commander le filtre de protection (2), **caractérisé en ce que** le détecteur d'UV utilisé se compose d'une couche fluorescente (F) et d'un photodétecteur au silicium (SD) associé, la couche fluorescente absorbant la lumière dans la plage des UV et en émettant dans la plage des ondes longues en fonction de la sensibilité spectrale du photodétecteur au silicium (SD).
